# EUROPEAN PATENT SPECIFICATION

(11) **EP 1 251 738 B1**
(45) Date of publication and mention of the grant of the patent: **17.12.2008**
(21) Application number: 01905454.3
(22) Date of filing: 05.02.2001
(51) Int. Cl.: A01N 37/10, C07C 233/54, C07C 59/64

(54) **NOVEL DIPHENYLETHYLENE COMPOUNDS**
NEUE DIPHENYLETHYLENE-VERBINDUNGEN
NOUVEAUX COMPOSES DE DIPHENYLETHYLENE

(30) Priority: 04.02.2000 US 180340 P; 17.08.2000 US 642618
(43) Date of publication of application: 30.10.2002
(73) Proprietor: Theracos, Inc., Sunnyvale, CA 94085 (US)
(72) Inventor: NAG, Bishwajit, Fremont, CA 94555 (US); DEY, Debendranath, Freemont, CA 94555 (US); MEDICHERLA, Satyanarayana, Cupertino, CA 95014 (US); NEOGI, Partha, Fremont, CA 94555 (US)
(74) Representative: Lipscombe, Martin John
(86) International application number: PCT/US2001/003797
(87) International publication number: WO 2001/056382

(56) References cited:
- WO-A-01/95859
- WO-A-02/02501
- WO-A1-00/69430
- WO-A1-99/50263
- JP-A- 9 059 245
- US-A- 2 524 827
- US-A- 5 430 062
- US-A- 5 525 632
- US-A- 5 589 506
- US-A- 5 686 478
- US-A- 5 827 898
- SETALA, HARRI ET AL: "A novel type of spiro compound formed by oxidative cross coupling of methyl sinapate with a syringyl lignin model compound. A model system for the .beta.-1 pathway in lignin biosynthesis" JOURNAL OF THE CHEMICAL SOCIETY, PERKIN TRANSACTIONS 1: ORGANIC AND BIO-ORGANIC CHEMISTRY , (4), 461-464 CODEN: JCPRB4; ISSN: 0300-922X, 1999, XP008048706
- DATABASE CA [Online] CHEMICAL ABSTRACTS SERVICE, COLUMBUS, OHIO, US; SPATH, ERNST ET AL: "Über die Inhaltsstoffe des roten Sandelholzes. III. Mitteil. : Die Synthese des Pterostilbens." XP008048672 retrieved from STN Database accession no. 1941:25276 & BER. , 74B, 189-92, 1941,
- PETTIT G.R. ET AL.: 'Isolation, structure, synthesis and antimitotic properties of combretastatins B-3 and B-4 from combretum caffrum' J. NAT. PROD. vol. 51, no. 3, 1988, pages 517 - 527, XP002938763

## Description

### Field of the Invention

The field of the invention is novel diphenylethylene compounds and their use for the treatment of diabetes and related conditions.

### Background of the Invention

Extracts of the leaves, flowers, and gum of the tree *Pterocarpus marsupium Roxb*. (*Leguminosae*), also known as the Indian Kino Tree, have been used traditionally to treat diarrhea, toothaches, fever, and urinary and skin infections. Extracts of the bark have been long regarded as useful for treating diabetes. Manickam et al. (J. Nat. Prod. 1997; 60:609-610) reported some hypoglycemic activity of a naturally occurring pterostilbene, trans-1-(3,5-dimethoxyphenyl)-2-(4-hydroxyphenyl)-ethylene, isolated from the heartwood of *Pterocarpus marsupium.* However, this pterostilbene is insoluble in water and has not been shown to be efficacious in the treatment of diabetes.

The causes of Type I and Type II diabetes are still unknown, although both genetic and environmental factors seem to be involved. Type I diabetes (or insulin-dependent diabetes) is an autoimmune disease in which the responsible autoantigen is still unknown. Subjects with Type I diabetes need to take insulin parenterally to survive. Type II diabetes (also referred to as non-insulin dependent diabetes mellitus, NIDDM) is a metabolic disorder resulting from the body's inability either to produce enough insulin or to properly' use the insulin that is produced. Insulin secretion and insulin resistance are considered the major metabolic defects, but the precise genetic factors involved remain unknown.

Subjects with diabetes usually have one or more of the following defects:
- Under-production of insulin by the pancreas
- Over-secretion of glucose by the liver
- Defects in glucose transporters
- Desensitization of insulin receptors
- Defects in metabolic breakdown of polysaccharides

In addition to insulin, which is administered parenterally, currently available medications used for diabetes include the 4 classes of oral hypoglycemic agents listed in the following table.

| | | | |
|---|---|---|---|
| **Class** | **Marketed Drugs** | **Mechanism of Action** | **Limitations** |
| Sulfonylureas | First generation: 2 Second generation: 3 | Signals beta cells to release more | Development of resistance |
| | | insulin | Hypoglycemia |
| Biguanides | Metformin | Reduces hepatic glucose | Improves sensitivity to insulin |
| | | production | Adverse hepatic effects |
| | | | Lactic acidosis |
| | | | Unwanted gastrointestinal effects |
| Glucosidase inhibitors | Acarbose | Reduces glucose absorption from gut | Works only after meals GI side effects |
| Thiazolidinediones | Troglitazone | Reduce insulin | Not effective in 25% of |
| | (withdrawn) Rosiglitazone Pioglitazone | resistance | subjects Require frequent liver function tests Have very long onset of action Cause weight gain |

As is apparent from the above table, there are disadvantages to the currently available antidiabetic agents. Accordingly, there is continuing interest in identifying and developing new agents-particularly orally administered, water-soluble compounds-that can be used to treat diabetes.

In addition to the pterostilbene discussed above, (-)-epicatechin has also been isolated from *Pterocarpus marsupium* by Sheehan et al. (J. Nat. Prod. 1983; 46:232) and reported as having a hypoglycemic effect (see also Chakravarthy et al. Life Sciences 1981; 29:2043-2047). Other phenolic compounds have been isolated from *Pterocarpus marsupium* by Maurya et al. (J Nat. Prod. 1984; 47:179-181), Jahromi et al. (J. Nat. Prod. 1993; 56:989-994), and Maurya et al. (Heterocycles 1982; 19:2103-2107).

WO 00/69430 discloses certain diphenylethylene and styrene compounds and their oral administration to decrease blood glucose levels in rats. The compounds are stated to be effective anti-diabetic agents.

### Summary of the Invention

In a first aspect, the present invention relates to the following compounds, namely: 3-(3,4-dimethoxy-phenyl)-2-(4-hydroxy-phenyl)-acrylic acid; 3-(3,5-dimethoxyphenyl)-2-(4-fluoro-p-phenyl)-acrylic acid; 2-(4-acetylamino-phenyl)-3-(3,5-dimethoxy-phenyl)-acrylic acid; or 3-(3,4-dimethoxy-phenyl)-2-(4-hydroxy-phenyl)-propionic acid.

In a second aspect, the present invention relates to a pharmaceutical composition for treatment of diabetes comprising a therapeutically effective amount of a compound, or a mixture of compounds, in accordance with the first aspect of the invention as defined above.

In a third aspect, the present invention relates to the use of a compound or a mixture of compounds in accordance with the first aspect of the invention as defined above, in the preparation of a medicament for treating diabetes.

### Brief Description of the Drawings

Figure 1 is a graph showing that compound la lowers blood glucose concentrations in rats with streptozotocin-induced diabetes.
Figure 2 is a graph showing that compound la lowers blood glucose concentrations in ob/ob mice.
Figures 3A, B, C, are graphs showing that compound la lowers insulin, triglyceride, and free fatty acid concentrations in ob/ob mice.
Figure 4 is a graph showing that compound la lowers blood glucose concentrations in db/db mice.
Figure 5A, B, C, are graphs showing that compound la lowers triglyceride and free fatty acid concentrations in db/db mice.
Figure 6 is a graph showing that compound la orally administered is more effective than IP administered in maintaining lowered blood glucose concentrations.
Figure 7 A, B are graphs showing that compound la lowers blood glucose concentrations in female obese (fa/fa) Zucker rats without affecting body weight.
Figure 8A, B, C, D are graphs showing that compound la improves the glucose tolerance of female obese fa/fa Zucker rats.
Figure 9 A, B are graphs showing that compound la lowers serum insulin, and increases leptin concentrations, in female obese Zucker fa/fa rats.
Figure 10 is a graph showing that compound la lowers cholesterol, triglyceride, and free fatty acid concentrations in female Zucker fa/fa rats.
Figure 11 A, B, C, D are graphs showing that compound la (20 mg/kg daily) lowers the insulin, triglyceride, free fatty acid, and cholesterol concentrations in male obese Zucker fa/fa rats.
Figure 12A, B are graphs showing that compound la does not lower glucose concentrations in normal animals.
Figure 13A, B are graphs showing that compound la stimulates glucose uptake in adipocytes.
Figure 14A, B, C, D are graphs showing that compound la increases GLUT-1 and GLUT-4 transporters in 3T3-L1 cells.
Figure 15A, B, C show, respectively, results of a lethal effect study on Swiss Webster mice by administration of compound la of dosages of 16.7, 167, and 333 mg/kg/BW on day zero.
Figure 16 is a graph showing that Wortmannin (a known PI-3 kinase inhibitor) blocks compound 1 a mediated glucose uptake in adipocytes.
Figure 17 is a graph showing compound la stimulates the phosphorylation of the insulin receptor β subunit and insulin receptor substrate 1 in CHO.IR cells.
Figure 18 is a graph showing compound la does not stimulate the phosphorylation of the IGF-1 receptor in CHO.IGF-1R cells.
Figure 19 is a graph showing that compound la stimulates the phosphorylation of Akt (protein kinase B) in CHO.IR cells.
Figure 20 is an illustration of a Western blot showing that Wortmannin inhibits compound la stimulated Akt phosphorylation.
Figure 21 is a graph showing that compound la does not up-regulate the expression of PPAR-γ in 3T3-L1 adipocytes.
Figure 22 summarizes the results of binding studies that show that compound la is not an agonist of nuclear PPARs.
Figure 23 is a graph showing compound la inhibits the binding of insulin to the insulin receptor.
Figures 24A, 24B are graphs showing that two isomers Ia and Ib (E and Z) stimulate rapid glucose uptake in rat adipocytes.
Figure 25A, B are graphs showing the results of pharmacokinetic studies of compound la in Sprague-Dawley rats.
Figure 26 is a chart summarizing the results of the toxicology studies conducted with compound la under Good Laboratory Practice regulations.

### Description of the Preferred Embodiments

Compounds in accordance with the present invention are provided by synthetic methods generally known in the art. See Pettit et al., J. Nat. Prod., 1988, 51 (3). pp 517-527 for a method for making E-isomers similar to compound la and Kessar et al., Indlan J. of Chem., 1981, 20B, pp 1-3 for making Z -isomers similar to compound lb (see below).

In general, compounds in accordance with the present invention may be prepared by the condensation of:
A) Appropriately substituted benzaldehyde or phenylketone with appropriately substituted phenylacetic acid or phenylacetic acid ester; B) Appropriately substituted benzaldehyde or phenylketone with appropriately substituted phenylacetamide; C) Appropriately substituted benzaldehyde or phenylketone with appropriately substituted phenylacetonitrile.

In Scheme I, the synthesis of compound la is shown as an exemplary synthesis. An exemplary synthesis of conversion of la to its Z-isomer is shown in Scheme II.

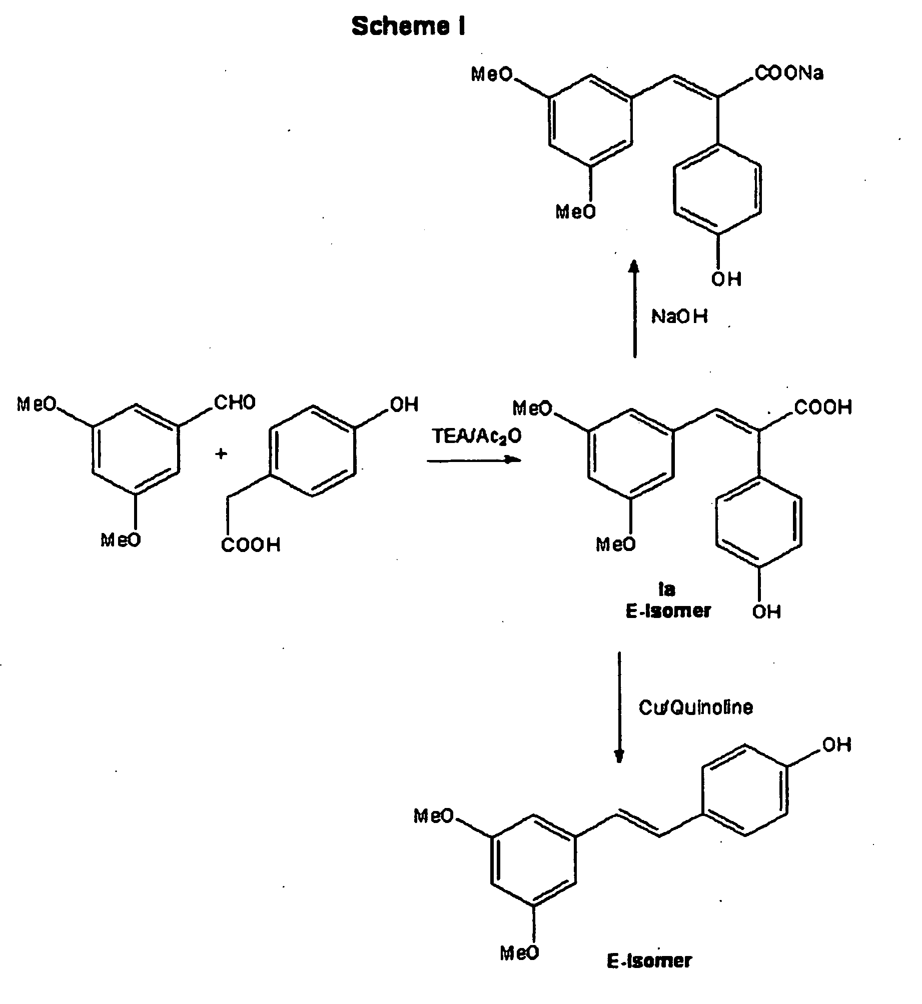

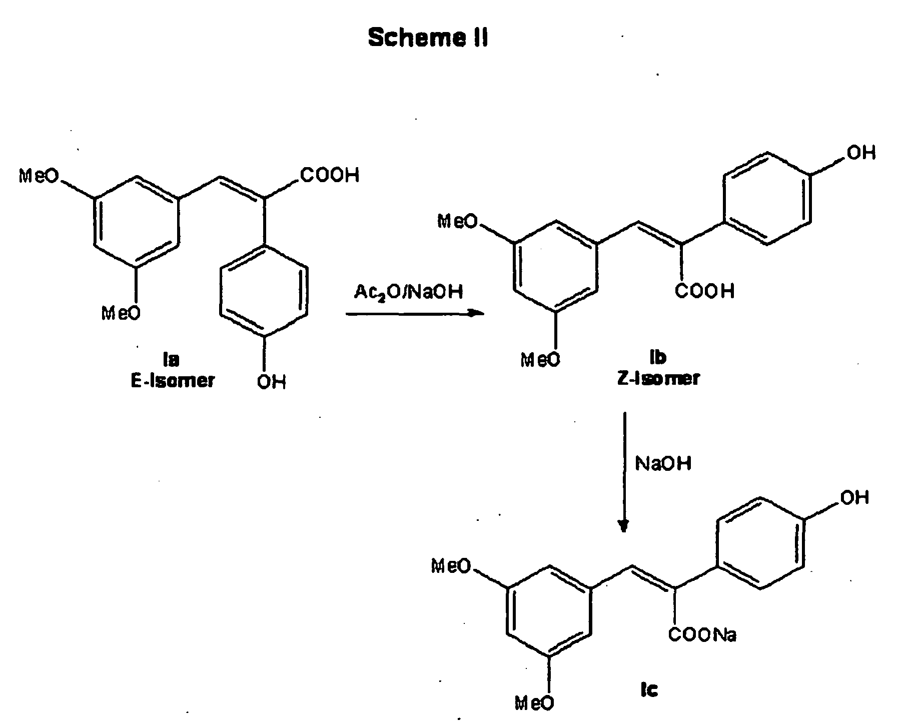

The compounds according to the present invention may be combined with pharmaceutically acceptable carriers and vehicles in various compositions suitable for oral or parenteral delivery. The particularly preferred form of composition is either an orally administered capsule or solution in which the compound is delivered in water, saline, or a phosphate buffer; or lyophilized powder in the form of tablets or capsules also containing various fillers and binders. The effective dosages of the compound in a composition will be selected by those of ordinary skill in the art and may be determined empirically.

The compounds of the present invention are useful for the treatment of diseases characterized by the presence of elevated blood glucose concentrations, i.e., hyperglycemic disorders such as diabetes mellitus, including both Type I and Type II diabetes, as well as other disorders related to hyperglycemia, such as obesity, increased cholesterol concentrations, and renal disorders.

"Treatment" means that the compound is administered at least to reduce the blood glucose concentration in the subject suffering from the hyperglycemic disorder; the compound may also reduce insulin or lipid concentrations or both. The compound is administered in an amount sufficient to reduce blood glucose concentration to an acceptable range, wherein an acceptable range means within about ± 10% of the normal average blood glucose concentration for a subject of that species. A variety of subjects in addition to humans may be treated with the compounds to reduce blood glucose concentrations, such as livestock, valuable or rare animals, and pets. The compounds may be administered to the subject suffering from the hyperglycemic disorder using any administration technique, including intravenous, intradermal, intramuscular, subcutaneous, or oral. However the oral route of administration is particularly preferred. The dosage delivered to the subject will depend on the route by which the compound is delivered, but generally ranges from 5 to 500 mg for a 70-kg human, typically about 50 to 200 mg for a 70-kg human.

Of particular interest are methods of treating human hyperglycemic disorders such as diabetes (both Type I and Type II) in which the compound is administered to the human suffering from the hyperglycemic disorder to at least reduce the blood glucose concentration of the subject to about the normal blood glucose range for a human; the compound may also reduce insulin or lipid concentrations or both.

The following examples are offered by way of illustration, and are not intended to limit the invention in any way.

### EXAMPLE 1

### Synthesis of E-3-(3,5-dimethoxy-phenyl)-2-( 4-hydroxy-phenyl)-acrylic acid (outside the scope of the present invention)

To a mixture of 3,5-dimethoxybenzaldehyde (30 mmol) and p-hydroxyphenyl acetic acid (30 mmol) was added 5 mL acetic anhydride and 2.5 mL of triethylamine (TEA). After being stirred at 130-140°C for 24 h, the mixture was cooled to room temperature and quenched with 25 mL concentrated HCl and extracted with CH₂Cl₂. The organic extract was further extracted with 1 N NaOH, then the NaOH extract was washed with CH₂Cl₂, and the aqueous layer was acidified with concentrated HCl and washed with water to obtain the crude product. Crude product was recrystallized from ethanol/water to yield the acid 1a.

Four lots of la (E-isomer) prepared as described above were separated in 40µl samples by HPLC on an Intersil ODS-3 (GL Sciences) column, 250 x 4.6 mm, and eluted with 62%v eluent A and 38%v eluent B. Eluent A is 0.1 % formic acid in water; B is 0.1 % formic acid in ACN. All samples showed a major amount of the E-isomer, with a minor amount of Ib (Z-isomer) at relative retention time 1.073±0.001. By this method, presence of the Z-isomer was estimated to be from 0.27% to 3.09% in these samples.

### Synthesis of Z-3-(3,5-dimethoxy-phenyl)-2-(4-hydroxy-phenyl)-acrylic acid (outside the scope of the present invention)

The Z-acid 1b was synthesized by a procedure described by Kessar *et al., supra,* who showed that E-a-phenyl cinnamic acids can be converted to similar Z-α-phenyl cinnamic acids by prolonged heating under basic conditions. The E-acid la (1.2 g, 4.0 mmol) was dissolved in a mixture of triethylamine (5.0ml) and acetic anhydride (0.5 ml) and heated to reflux for 24 hours. The mixture was then cooled, diluted with ethyl acetate, and extracted sequentially first with 5% HCl (aqueous) then with 2 N NaOH and water. The combined basic aqueous solutions were acidified to a pH of 5 with acetic acid and cooled, and the solid was filtered. The filtrate was further acidified with concentrated HCl. Precipitation occurred upon cooling. The solid was collected by filtration and washed with fresh water. The solid compound was air dried to yield 1b.

Both isomers were subjected to NMR, pKa, HPLC, and UV spectral analysis.

**E-Isomer.** The free acid form of the E-isomer showed a chemical shift for the olefinic proton (in DMSO-d₆) of δ7.59. The free acid has a melting point of 225-227°C and a pKa of 6.2.

**Z-Isomer.** The ¹H NMR analysis of the Z-isomer produced as described above showed the chemical shift of the olefinic proton to be δ6.81 as a free acid in DMSO-d₆. The free acid form has a melting point of 135-137°C and a pKa of 5.3.

**Comparison of Isomers Produced.** The chemical shifts of the olefinic protons of the E- and Z- isomers prepared as described above are δ7.59 and δ6.81, respectively. As reported by Gadre and Marathe, Synth Commun 1988; 18: 1015-1027, the compound with the higher chemical shift of the olefinic proton is the E-isomer, and the respective shifts seen with the prepared compounds are in agreement with that.

The analysis of the Perkin reaction product of phenyl acetic and benzaldehyde (a similar compound), indicates that the pKa of the isomers of α-phenyl cinnamic acid are 6.1 for the E- isomer and 4.8 for the Z-isomer, Fieser LF and Williamson KL, Exp. In Org. Chem (3rd ed.), Lexington, MA; Heath and Company, 1955, p 182. Accordingly, between the two isomers, the one having the higher pKa is the E- isomer.

### HPLC AND UV Spectral Analysis

The reverse-phase HPLC analysis of E- and Z- isomers was performed by a linear gradient using a 0.1 % formic acid/water/acetonitrile system on a G.L. Sciences Intersil ODS-3 column (250 x 4.6 mm, 5 µm), monitored at 280 nm. In this system, the E- and Z- isomers were eluted at 17.4 and 17.9 min, respectively.

Each isomer has a distinct UV spectrum. The λmax values for the E- isomer are 227 nm and 284 nm, and those for the Z- isomer are 221 nm and 303 nm.

### Synthesis of E-4- [2 -(3,5 -dimethoxy-phenyl)-vinyl]- phenol (outside the scope of the present invention)

To decarboxylate la, 3 g of Cu powder and 30 mL of quinoline were added to 1 g of la under N₂ and refluxed with stirring for 4 h (still under N₂). The reaction mixture was filtered, acidified with concentrated HCl, and extracted with CH₂Cl₂. The organic layer was washed with aqueous saturated NaCl, dried and concentrated. The decarboxylated product was purified by flash chromatography over silica gel.

### Synthesis of E-3-(3,5-dimethoxy-phenyl)-2-(4-hydroxy-phenyl)-acrylic acid sodium salt (outside the scope of the present invention)

To convert the acid la to the sodium salt, NaOH solution was added to 1 g of la under room temperature; the mixture was shaken and freeze-dried to give the sodium salt of la.

### EXAMPLE 2

### Synthesis of 3-(3,4-Dimethoxy-phenyl)-2-(4-hydroxy-phenyl)-acrylic acid

To a mixture of 3,4-dimethoxybenzaldehyde (9.97g, 60mmol) and p-hydroxyphenyl acetic acid (10.0g, 65mmol) under argon atmosphere was added acetic anhydride (12mL) and triethylamine (8.0mL, 58mmol). The mixture was stirred at 140°C for 18h. The reaction mixture was cooled to 5°C and dichloromethane (100mL) was added. To this yellow suspension concentrated HCl (20 ml) was added and the suspension stirred for 20min. The solid separated was filtered, dissolved in aqueous sodium hydroxide (2M, 225mL) and re-precipitated with concentrated HCl (40mL). Yellow solid was filtered and washed with water (2x30mL) and the wet solid was recrystallized from a water-ethanol mixture.
¹H NMR (DMSO-d₆): δ12.38 (br. 1 H), 9.47 (br, 1 H), 7.61 (s, 1 H), 6.96 (d, J=8.4 Hz, 2H), 6.81-6.77 (overlapped, 4H), 6.54 (br, 1 H), 3.40 (s, 3H) and 3.37 (s, 3H).

### Synthesis of 3-(3,5-Dimethoxy-phenyl)-2-(4-fluoro-p-phenyl)-acrylic acid

To a mixture of 3,5-dimethoxybenzaldehyde (4.98g, 30mmol) and p-fluorophenyl acetic acid (4.62g, 30mmol) under argon atmosphere was added acetic anhydride [5mL) and triethylamine (5.0ml, 36mmol). The mixture was stirred at 140°C for 18h. The reaction mixture was cooled to room temperature and diethyl ether (100mL) was added. The ether solution was further cooled to 10°C and acidified with concentrated HCl (35mL). The aqueous layer was discarded and the organic layer was extracted with aqueous sodium hydroxide solution (2M, 3x75mL). Aqueous layers were pooled together and acidified with concentrated HCl (40mL). The resulting precipitate was filtered, washed with water (2x30mL) and re crystallized from a water-ethanol mixture.
¹H NMR (DMSO-d₆): δ 12.73 (br, 1 H), 7.69 (s, 1 H), 7.22 (d, J=7.2 Hz, 4H), 6.38 (t, J=2.5Hz, 1H), 6.23 (d, 2.5Hz, 2H), and 3.33 (s, 6H).

### Synthesis of 2-(4-Acetylamino-phenyl)-3-(3,5-dimethoxy-phenyl)-acrylic acid

To a mixture of 3,5-dimethoxybenzaldehyde (2.5g, 15mmol) and p-aminophenyl acetic acid (2.28g, 15mmol) under argon atmosphere was added acetic anhydride (5mL) and triethylamine (3.4mL, 24mmol). The mixture was stirred at 140°C for 2h. The reaction mixture was cooled to room temperature and chloroform (50mL) was added. The chloroform solution was further cooled to 10°C and acidified with concentrated HCl (10mL). The aqueous layer was discarded and the organic layer was extracted with aqueous sodium hydroxide solution (2M, 3x50mL). Aqueous layers were pooled and acidified with concentrated HCl to pH 1. The resulting precipitate was filtered, washed with water (2x30mL) and recrystallized from a water-ethanol mixture.
¹H NMR (DMSO-d₆): δ 12.70 (br, 1 H), 10.04 (s, 1 H). 7.63 (s, 1 H), 7.54 (d, J=7.5 Hz, 2H), 7.08 (d, J=7.5 Hz, 2H), 6.36(t, J=2.4Hz, 1 H), 6.25(d, J=2.4Hz, 2H), 3.56(s, 6H) and 2.04(s, 3H).

### Synthesis of 3-(3,4-Dimethoxy-phenyl)-2-(4-hydroxy-phenyl)-propionic acid

3-(3,4-Dimethoxy-phenyl)-2-(4-hydroxy-phenyl)-acrylic acid was dissolved in ethanol (100mL) and palladium-charcoal (10%, 50% wet, 0.3g) was added. The mixture was stirred overnight under hydrogen at room temperature. The reaction mixture was filtered through a bed of Celite® diatomaceous earth and the solvent evaporated.
¹H NMR (DMSO-d₆): δ 12.15 (br, 1H), 9.22 (br, 1H), 7.10 (d, J=8.6 Hz, 2H), 6.67 (d, J=8.6 Hz, 2H), 6.31 (d, J=2.2Hz, 2H), 6.27 (t, J=2.2Hz, 1H), 3.70 (s, 6H), 3.14 (dd, J=13.3 and 8.6Hz. 2H), and 2.80 (dd, J=13.3 and 8.6Hz, 2H).

### Example 3 (outside the scope of the present invention)

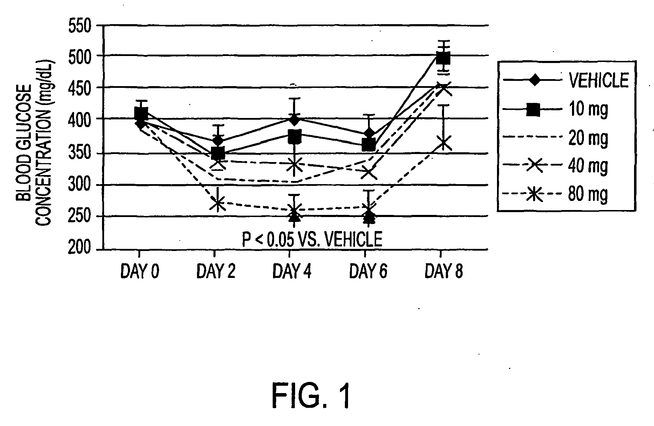

Diabetes was induced in Sprague-Dawley (SD) male rats (average body weight, 180 g) by IV injection of 60 mg streptozotocin. After 5 days, the average glucose concentration was in the range of about 350-400 mg/dL. Rats were then divided into five groups (n = 7) and given either phosphate-buffered saline (PBS) (vehicle) or compound la (10,20,40 or 80 mg/kg of body weight) orally daily for 8 days. Treatment with la at a dose of 20, 40 or 80 mg/kg reduced the blood glucose concentrations of these rats (compared to those of vehicle-treated controls) from Day 2 through Day 6. The reduction was statistically significant (p < 0.05) at Day 4 and Day 6 of the rats given 80 mg/kg. Results are shown in FIG. 1.

### Example 4 (outside the scope of the present invention)

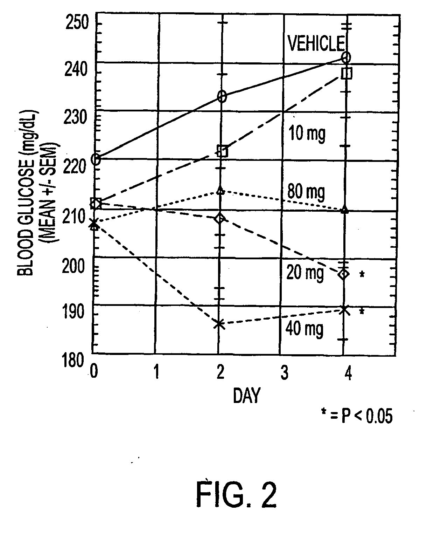

Obese (ob/ob) mice spontaneously develop diabetes, with glucose concentrations ranging between 200 and 300 mg/dL. In this experiment, la was administered daily in doses of 0 (vehicle only), 10, 20, 40, or 80 mg/kg of body weight to ob/ob mice for 4 days. By Day 4, the blood glucose concentrations or the animals given la were lower than those of the animals given the vehicle only; the differences were statistically significant for the groups given 20 and 40 mg/kg. Results shown in FIG. 2.

### Example 5 (outside the scope of the present invention)

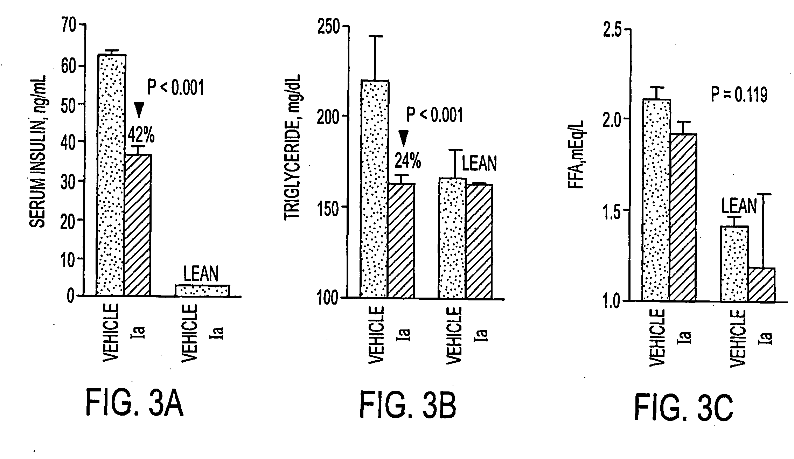

Serum concentrations of insulin, triglyceride, and free fatty acids (FFA) in ob/ob mice were measured on Day 8 following daily oral treatment with a dose of 20 mg/kg body weight for 7 days. Serum insulin concentrations were 42% lower in the la treated animals than they were in the vehicle-treated animals (A). Serum triglyceride concentrations were 24% lower in the la-treated mice than in the vehicle-treated mice (B). Serum FFA concentrations did not decrease significantly (C). Results shown in FIGS. 3A, B, C.

### Example 6 (outside the scope of the present invention)

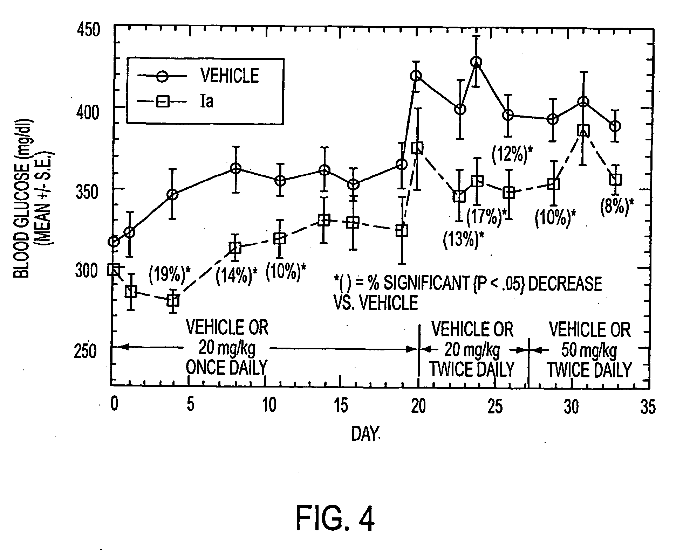

The ability of la to reduce glucose concentrations was examined further in db/db mice. Eight-week-old db/db mice with average blood glucose concentrations of 280-300 mg/dL were treated with vehicle or la (single 20-mg/kg doses daily, for 20 days; two 20-mg/kg doses daily for 8 days; and two 50-mg/kg doses daily for 5 days). Blood glucose concentrations in the mice given la were reduced 20% from those in the mice given the vehicle at the end of the first 20 days of treatment. Treatment with la at higher doses did not improve the glucose-lowering effect. Results shown in FIG. 4.

### Example 7 (outside the scope of the present invention)

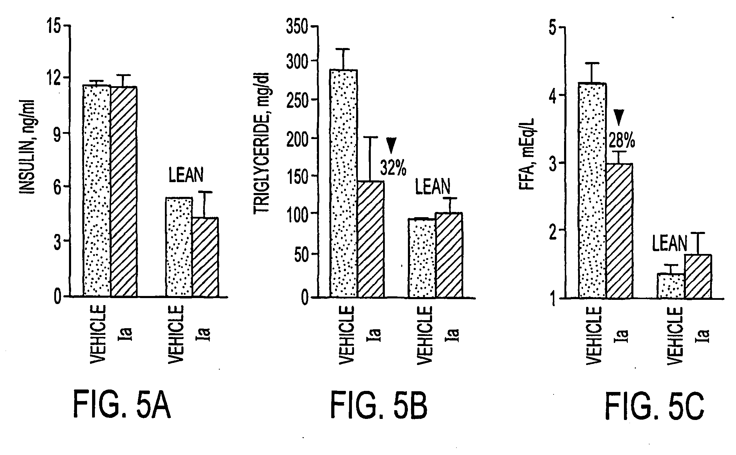

FIGS. 5A, B, C show the serum insulin, triglyceride, and free fatty acid (FFA) concentrations found in the db/db mice treated with la from the experiment shown in Figure 4 (the analyses were done at the end of the experiment). Although the insulin concentrations in the 1A-treated and vehicle-treated groups did not differ (A), the triglyceride (B) and FFA (C) concentrations were significantly lower in the mice treated with la than they were in the mice treated with vehicle, triglyceride concentrations were reduced 32% from those in the vehicle-treated mice, and free fatty acid concentrations were reduced 28% from those in the vehicle-treated mice.

### Example 8 (outside the scope of the present invention)

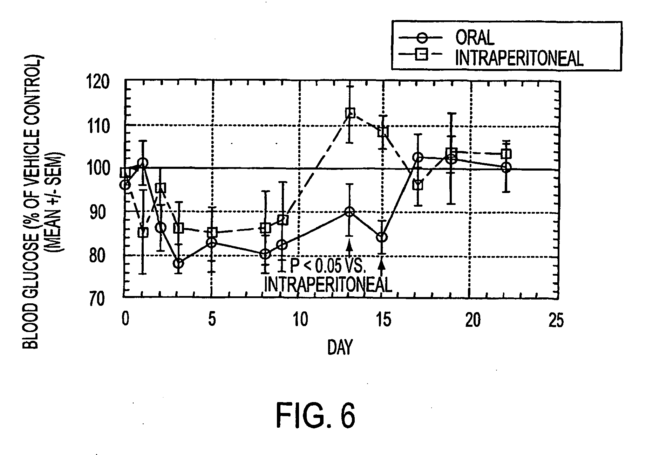

Compound la was administered either orally or intraperitoneally (IP) at a dose of 20 mg/kg to db/db mice for 22 days. After Day 9, the glucose-lowering effect of IP administered la disappeared, but that of orally administered la was maintained. The differences between the mice given oral la and those given IP la were statistically significant (p < 0.05) on Days 13 and 15. Results shown in FIG. 6.

### Example 9 (outside the scope of the present invention)

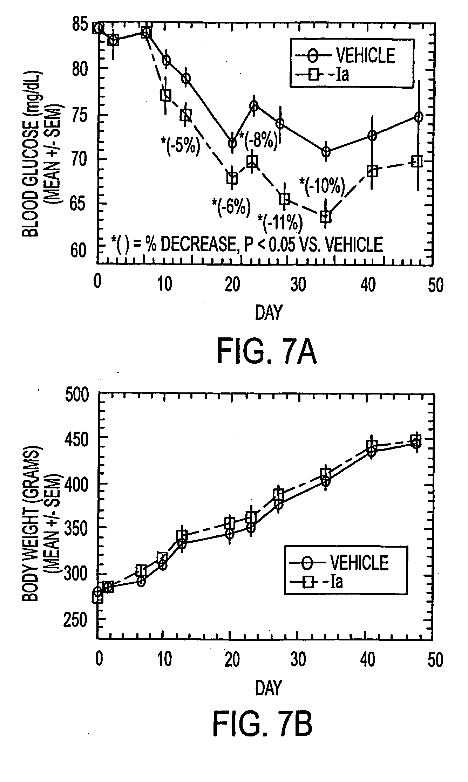

The effect of la was studied in female Zucker (fa/fa) rats (considered a good spontaneous genetic model of investigating insulin-resistant diabetes). Female fa/fa rats were given vehicle or la (20 mg/kg) daily for 58 days. (A) The blood glucose concentrations of the rats given la were lower than those of the rats given vehicle from Day 10 through the end of the experiment, and the differences were statistically significant on Days 9 through 34. (B) Throughout the experiment, the body weights of the rats in the two treatment groups were virtually identical. Results shown in FIGS. 7 A, B.

### Example 10 (outside the scope of the present invention)

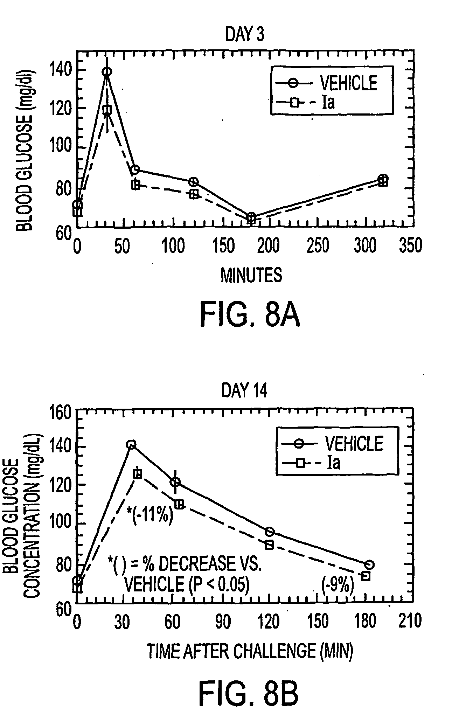

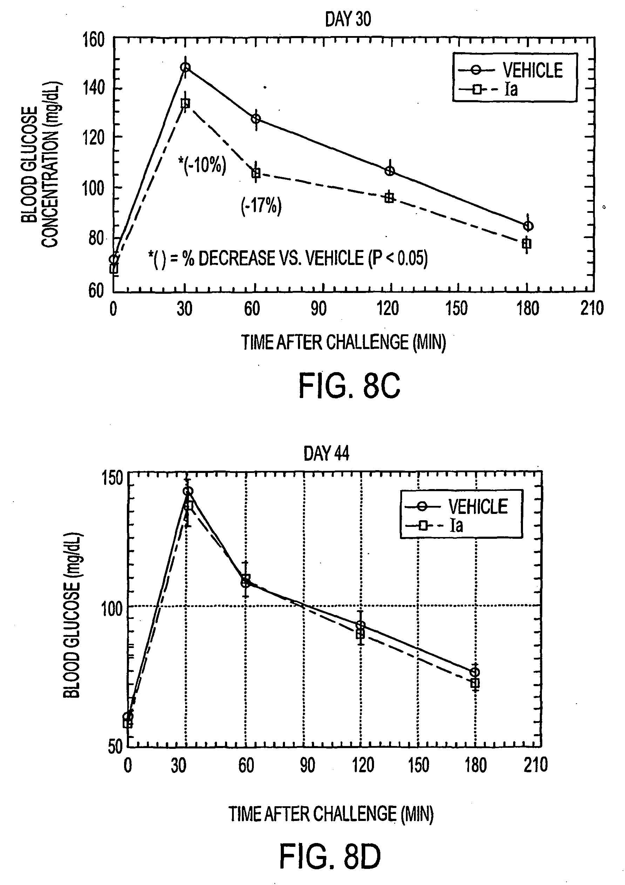

Zucker fa/fa rats were given vehicle or la (20 mg/kg) daily for 58 days; on Days 3, 14, 30, and 44, glucose tolerance tests (glucose, 2 mg/kg in water) were administered. The results of these tests show that the differences between treatment groups were statistically significant (p < 0.05) 30 and 180 minutes after challenge on Day 14 (B) and 30 and 60 minutes after glucose challenge on Day 30 (C). By Day 44, the effect of la on glucose tolerance had disappeared. Results shown in FIGS. 8A-D.

### Example 11 (outside the scope of the present invention)

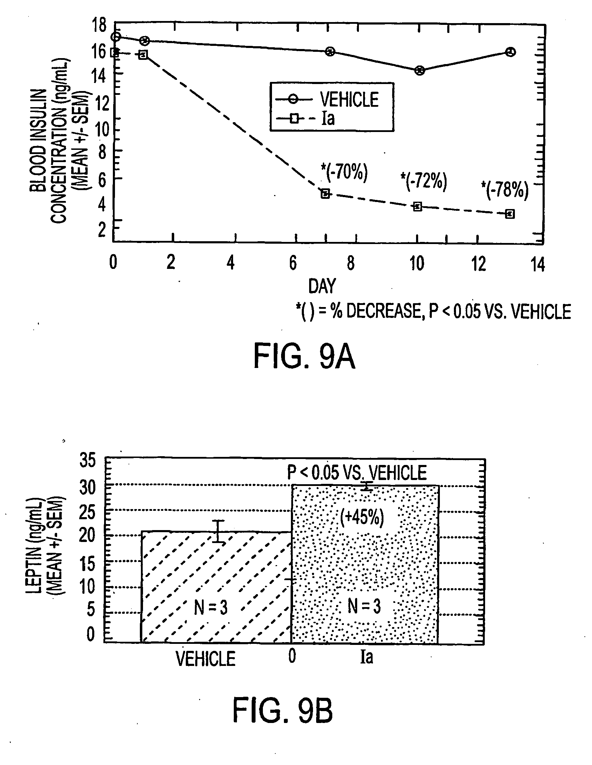

(A) The Zucker fa/fa rats treated with la (20 mg/kg) for 58 days (see Figure 7) had serum insulin concentrations that were decreased 70-78% from those in the rats given vehicle only (p < 0.05). This suggests that the mechanism by which la affects diabetes involves insulin-sensitization.
(B) In addition, the serum leptin concentrations of the IA-treated rats were 45% higher than those of the vehicle-treated rats. Results shown in FIGS. 9A, B.

### Example 12 (outside the scope of the present invention)

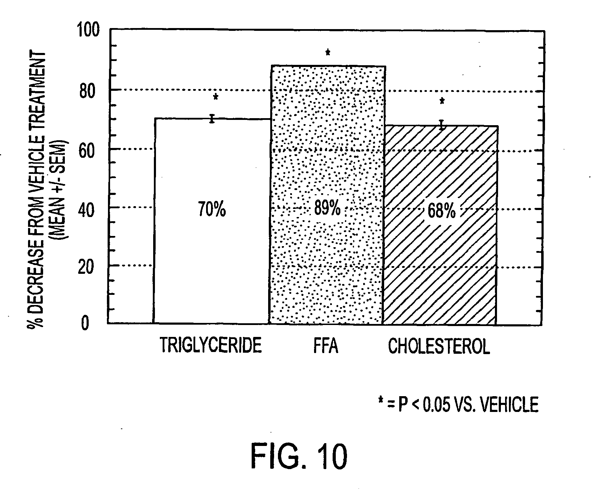

Serum concentrations of triglyceride, free fatty acid, and cholesterol were also measured in the Zucker fa/fa rats given vehicle or la (20 mg/kg) daily for 58 days (see also Figures 7 and 9). At the end of the study, the triglyceride, free fatty acid, and cholesterol concentrations found in the rats given la were reduced 70%, 89%, and 68% from those of the rats given vehicle. Results shown in FIG. 10.

### Example 13 (outside the scope of the present invention)

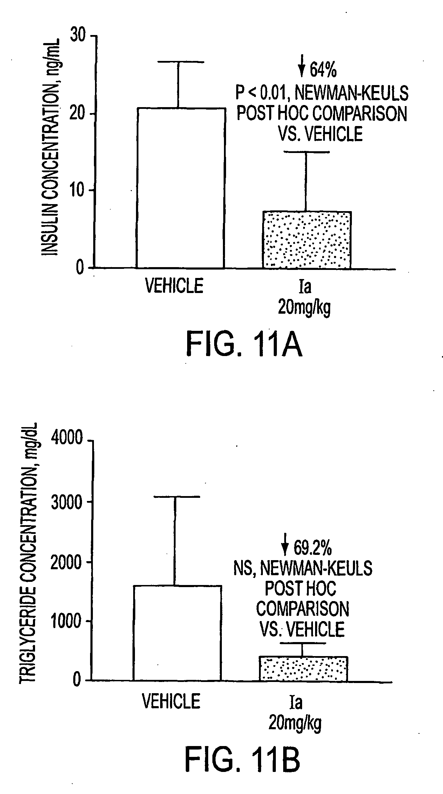

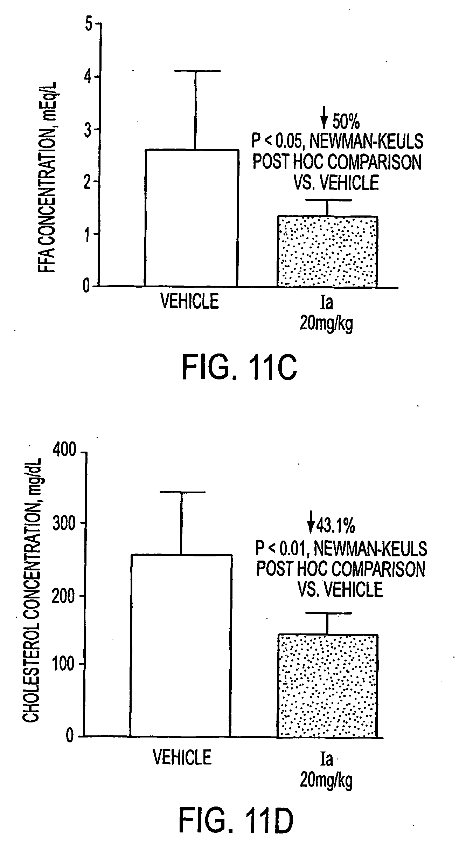

When the test described in connection with Figures 7-10 was conducted using male obese Zucker fa/fa rats treated with vehicle or la (20 mg/kg) daily for 65 days, the glucose concentrations, glucose tolerance, and leptin concentrations of the la-treated animals did not differ from those of the vehicle-treated animals. However, the insulin, triglyceride, free fatty acid, and cholesterol concentrations found in the la-treated rats at the end of the experiment were all lower than those found in the vehicle-treated animals. Results shown in FIG. 11 A-D.

### Example 14 (outside the scope of the present invention)

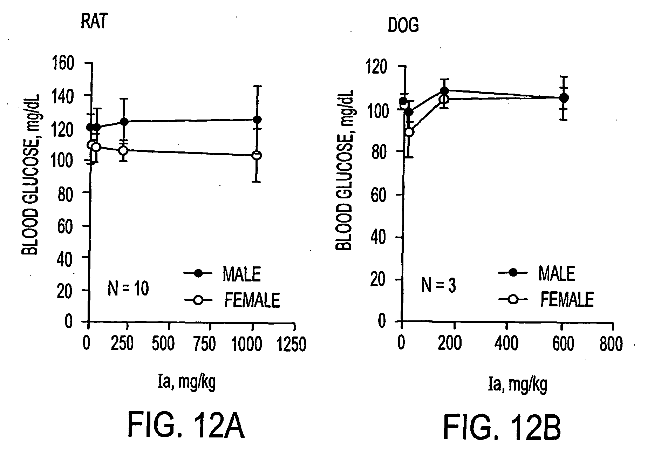

Compound la does not lower blood glucose concentrations in normal animals. This was demonstrated in two studies using rats and dogs. Daily oral administration of la for 28 days did not cause any hypoglycemic activity of this compound, even at very high doses (up to 1000 mg/kg). Results shown in FIG. 12A, B.

### Example 15 (outside the scope of the present invention)

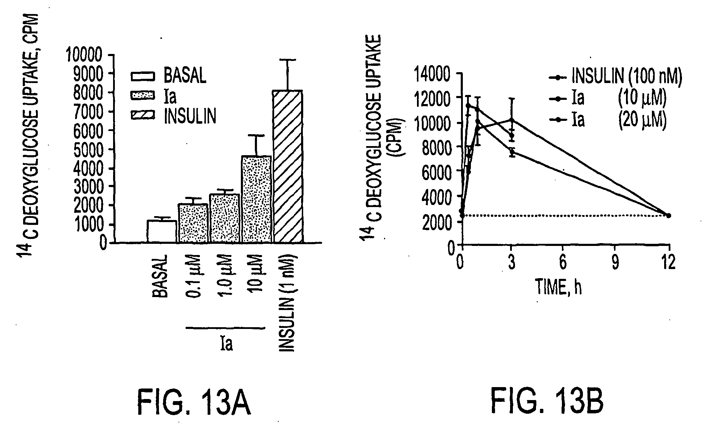

Glucose uptake was measured in normal adipocytes freshly prepared from the epididimal fat pad of Sprague-Dawley (SD) rats (170 g) in the presence of la at the indicated concentrations (A), and in differentiated 3T3-L1 adipocytes (B). Insulin was used as a positive control in both experiments. In both cases la stimulated glucose uptake in a manner similar to insulin. Results shown in FIGS 13A, B.

### Example 16 (outside the scope of the present invention)

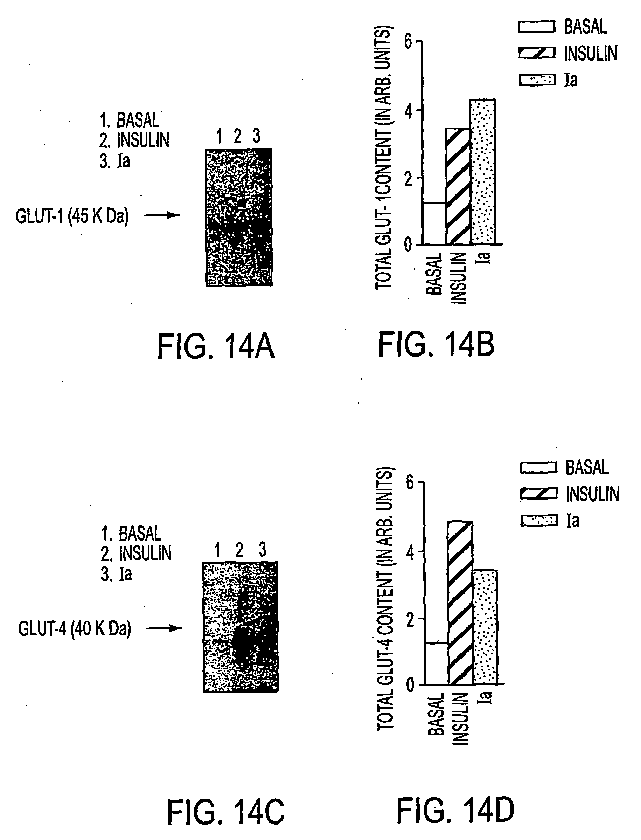

To determine whether la treatment affected expression of glucose transporters GLUT-1 and GLUT-4, differentiated 3T3-L1 adipocytes were treated with la, insulin or vehicle alone. Cells were lysed, subjected to 4-20% gradient SDS-PAGE, electroblotted, and probed with anti-GLUT-1 or anti-GLUT-4 monoclonal antibodies. As this figure shows, both GLUT-1 (A) and GLUT-4 (B) were up-regulated in 3T3-L 1 cells following exposure of cells to la. Results shown in FIGS. 14A-D.

### Example 17 (outside the scope of the present invention)

Differentiated 3T3-L 1 adipocytes were serum starved for 3 hours and then treated with either vehicle (medium alone) or la at a concentration of 10 µM for 30 minutes at 37°C. Cells were washed with phosphate-buffered saline (PBS), fixed with methanol at -20°C for 20 minutes, rinsed with PBS three times, and incubated with PBS containing 10% calf serum for 30 min at 37°C. The slides were incubated with anti-GLUT-4 polyclonal antibody (1 :50 dilution) in 10% calf serum for 2 hours at 37°C. Following this incubation, the slides were rinsed with PBS three times and then incubated with secondary antibody coupled with Alexa-Fluor (EXₘₐₓ 495 nm; Emₘₐₓ 519 nm) for 30 min. Finally, the slides were rinsed with PBS and mounted with prolonged antifade mounting media. The pictures generated using a Nikon confocal PCM 2000 microscope linked to an image analyzer showed high fluorescence in the la-treated cells. The fluorescence staining appeared in the cell membrane, indicating that treatment with la promoted translocation of GLUT-4 glucose transporters to the cell surface.

### Example 18 (outside the scope of the present invention)

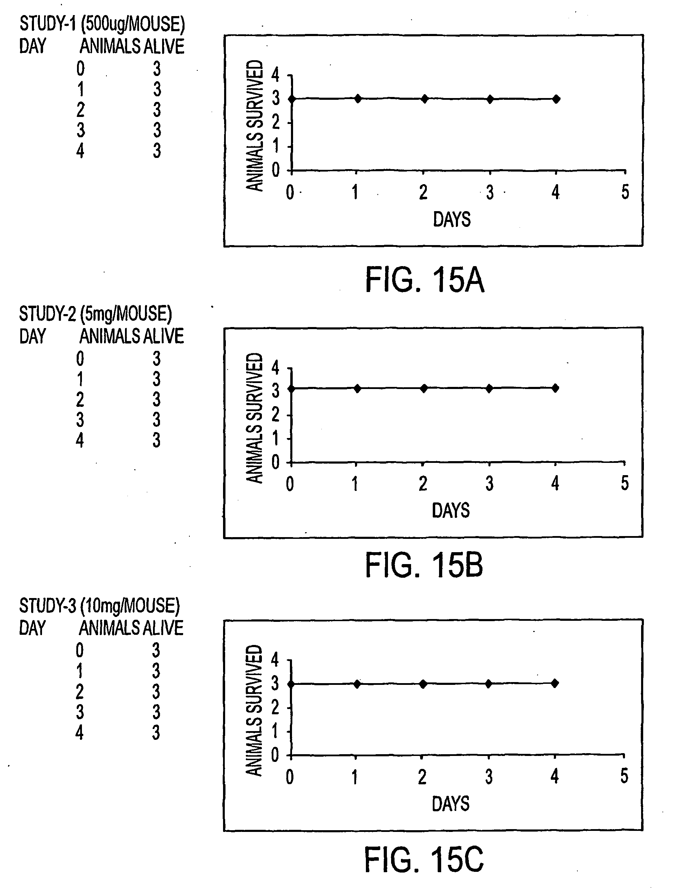

Nine healthy male Swiss Webster mice were divided into three study groups of three. The first study group (FIG. 15A) received the compound of la at a dose of 16.7 mg/kgBW, the second study group (FIG. 15B) received a dose of 167 mg/kg/BW, and the third study group (FIG. 15C) received a dose of 333 mg/kgBW on day zero of the study. The mice were kept on regular food and water during the entire study period. During the study, the mice were under close observation and their behaviour, gross physiology and mortality/survival were monitored. FIGS. 15A, 15B and 15C show that the survival rate in these mice in the course of the study period was 100%.

### Example 19 (outside the scope of the present invention)

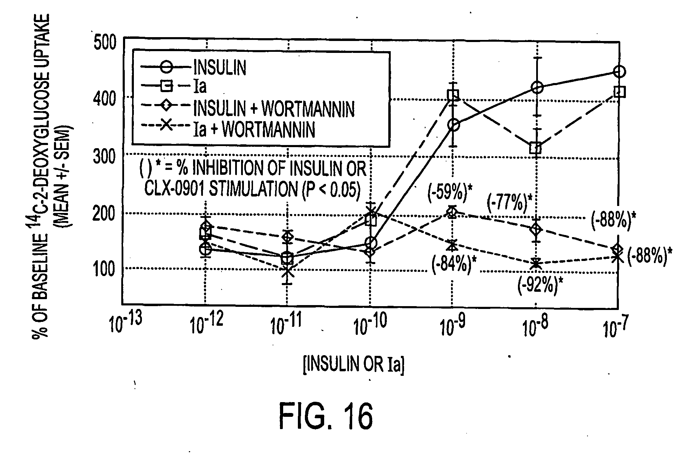

Wortmannin is a known inhibitor of phosphatidylinositol 3-kinase (PI 3-kinase), an enzyme required for the insulin-signaling pathway. In this experiment, the ability of wortmannin to inhibit la-stimulated glucose uptake was measured. Freshly prepared adipocytes were incubated with varying concentrations of either insulin or la, in the presence or absence of 4 µM wortmannin. The ability of adipocytes to take up glucose was then monitored using the ¹⁴C-deoxyglucose tracer. As shown here, treatment of adipocytes with wortmannin strongly inhibits insulin or la-dependent glucose uptake. This result suggests that la influences the PI3-kinase pathway. Results shown in FIG. 16.

### Example 20 (outside the scope of the present invention)

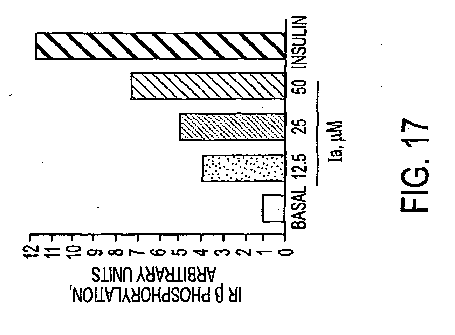

Chinese hamster ovary cells that overexpress the human insulin receptor (CHO.IR cells) were grown in F12 Ham's medium with 10% fetal bovine serum (FBS) at 37°C in 5% CO₂. Cells were serum-starved for 6 hours, and then incubated with vehicle, insulin (10 nM), or la (12.5, 25, or 50 µM) for 30 minutes at 37°C. Then the cells were washed with cold phosphate-buffered saline (PBS), and 13µg of total cell lysates were separated by electrophoresis (4-20% SDS-PAGE), blotted onto a nitrocellulose membrane, and phosphorylation was detected with monoclonal antibody to phosphotyrosine (Transduction Laboratories, clone PY20). Western blots were developed using an enhanced chemiluminescence detection system, and the results were quantified by scanning then expressed as arbitrary units. The results indicate that la phosphorylates the insulin receptor and insulinreceptor substrate 1 in a dose-dependent manner (as does insulin). Results shown in FIG. 17.

### Example 21 (outside the scope of the present invention)

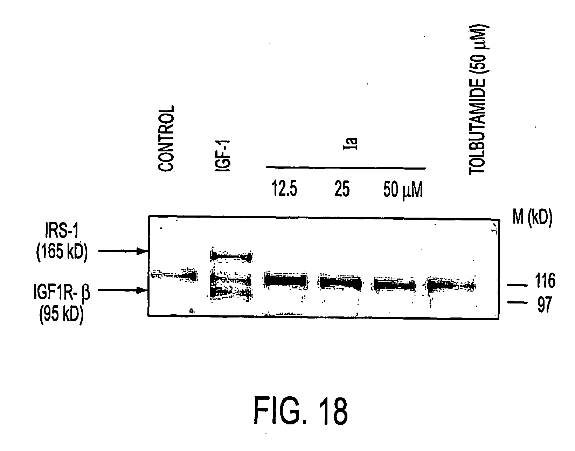

Chinese hamster ovary cells that overexpress the human insulin-like growth factor 1 receptor (CHO.IGF-IR cells) were grown in F12 Ham's medium with 10 % FBS at 37°C in 5% CO₂. Cells were serum-starved for 6 hours, and then incubated with vehicle, IGF-1 (100 nM), tolbutamide (50µM], or la (12.5, 25, or 50µM) for 30 minutes at 37°C. Then the cells were washed with cold phosphate-buffered saline (PBS), and 13µg of total cell lysates were separated by electrophoresis (4-20% SDS-PAGE) and blotted onto a nitrocellulose membrane; phosphorylation was detected with monoclonal antibody to phosphotyrosine (Transduction Laboratories, clone PY20). The results show that la does not phosphorylate the insulin-like growth factor 1 receptor or insulin receptor substrate 1 in CHO.IGF-1R cells. Results shown in FIG. 18.

### Example 22 (outside the scope of the present invention)

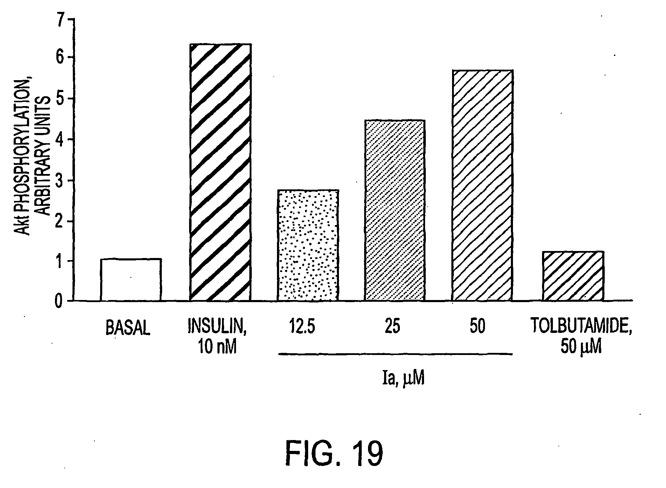

Chinese hamster ovary cells that overexpress the human insulin receptor (CHO.IR) were grown in F12 Ham's medium with 10% fetal bovine serum (FBS) at 37°C in 5% CO₂. Cells were serum starved for 6 hours and incubated with vehicle, insulin (10 nM), tolbutamide (50 µM), or one of 3 different doses of la (12.5,25, or 50µM) for 30 min at 37°C. Then the cells were washed with cold phosphate-buffered saline (PBS), and 25µg of total cell lysates were separated by electrophoresis (4-20% SDS-PAGE), blotted onto a membrane, and detected with the antibody (A) anti Phospho-Akt (Ser 473) (New England Biolabs). Western blots were developed using an enhanced chemiluminescence detection system, and the results were quantified by scanning and then expressed as arbitrary units. The results indicate that there was a dose-dependent increase in phosphorylation of Akt in the presence of la. Results shown in FIG. 19.

### Example 23 (outside the scope of the present invention)

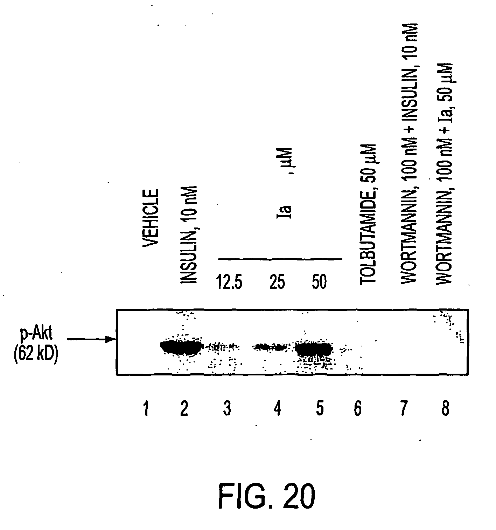

Chinese hamster ovary (CHO) cells that had been serum-starved for 6 h were incubated with vehicle, insulin (10 nM), tolbutamide (50 µM), or one of three doses of la (12.5, 25, or 50µM) for 30 minutes at 37°C. Two groups had been, preincubated with 100 nM Wortmannin (Lanes 7 and 8), and had the insulin (10 nM) and la (50µM) added at this time. Then cells were washed with cold phosphate-buffered saline (PBS), and 20 µg of total cell lysates were separated by electrophoresis (4-20% SDS-PAGE) and blotted onto a membrane; Akt-phosphorylation was detected with an antibody [anti Phospho-Akt (Ser 473), New England Biolab]. The results show that Wortmannin inhibited the Akt-phosphorylation stimulated by insulin and by la. Results shown in FIG. 20.

### Example 24 (outside the scope of the present invention)

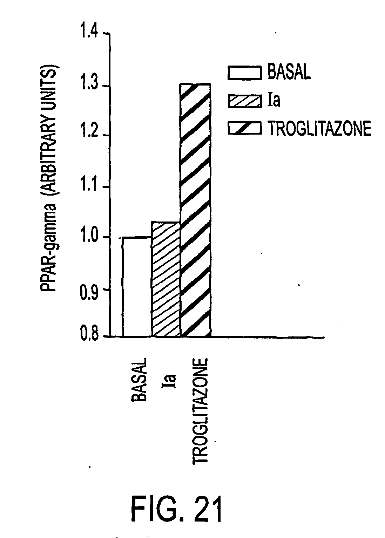

All thiazolidinedione compounds are known to stimulate glucose uptake via a mechanism that involves binding to and increasing the expression of a nuclear receptor transcription factor known as peroxisome proliferator activated receptor-γ (PPAR-γ). To determine whether la up-regulates glucose uptake by a mechanism that involves PPAR-γ, 3T3-L 1 adipocytes were incubated with vehicle, la (5 µM), or troglitazone (5 µM), for 48 hours. PPAR-γ expression was assessed by immunoblotting. Western blots were developed using an enhanced chemiluminescence detection system, and the results were quantified by scanning and then expressed as arbitrary units. The results in the figure show that troglitazone induced an increase in PPAR-γ, while la did not induce an increase in PPAR-γ over the basal level of this transcription factor. Results shown in FIG. 21.

### Example 25 (outside the scope of the present invention)

Differentiation of fibroblasts to adipocytes involves the expression of PPAR-γ. All members of the thiazolidinedione class of antidiabetic compounds stimulate PPAR-γ expression and promote the differentiation of fibroblasts to adipocytes. Similarly, insulin also stimulates the differentiation of fibroblasts to adipocytes. To examine the effect of la on this differentiation process, 3T3-11 fibroblasts were incubated with la (1 µM), insulin (0.17 mM) or a combination of both. Following incubation, the cells were lysed, and the quantity of expressed PPAR-γ was analyzed by ECL blot analysis using anti-PPAR-γ antibody. Treatment of fibroblasts with la did not enhance the differentiation process. In a positive control, insulin treatment of fibroblasts stimulated the differentiation of these cells to adipocytes in association with increased levels of PPAR-γ.

### Example 26 (outside the scope of the present invention)

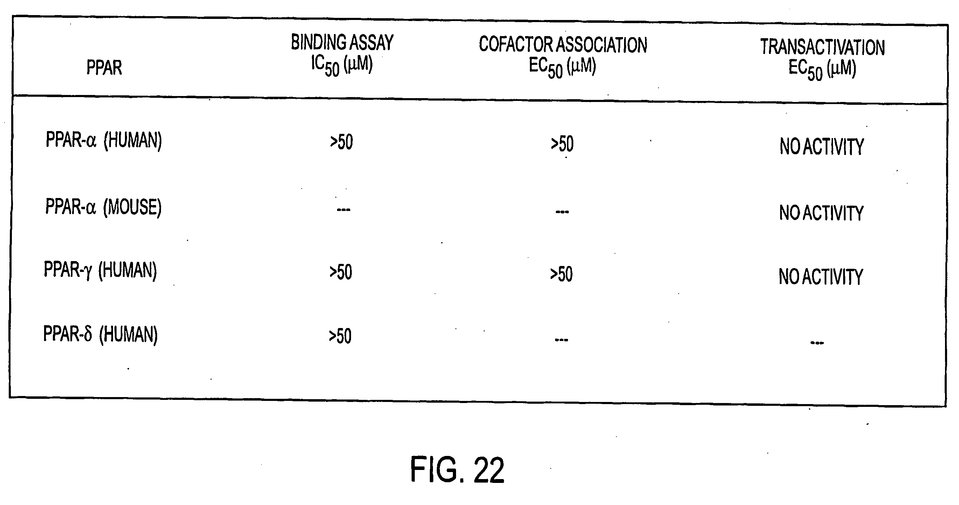

FIG. 22 shows the results of three tests conducted to determine whether or not la is an agonist of nuclear PPAR receptors. The ability of la to bind human recombinant PPAR-α, PPAR-γ, or PPAR-δ was shown using a radioligand-binding assay that measures the displacement of an established radiolabeled ligand. In this assay, the IC, values for all three nuclear receptors were greater than 50 µM. Ligand induced conformational changes in PPAR ore known to promote the binding of coactivator molecules. The cofactor association was measured by the time-resolved fluorescence (HTRF) assay that uses energy transfer between two adjacent molecules to measure the ability of la to promote the association of PPARs with cofactor proteins. la did not induce any association of cofactors. Finally, a cell-based transactivation functional assay was performed to determine the effect of la on PPARs in a biological system. In this experiment, COS cells with chimeric receptors were treated with la, and the transcription activity was measured by an increase in luciferase activity. No activation of PPARs by la was observed. All of these results confirm that la is not an agonist of these PPARs.

### Example 27 (outside the scope of the present invention)

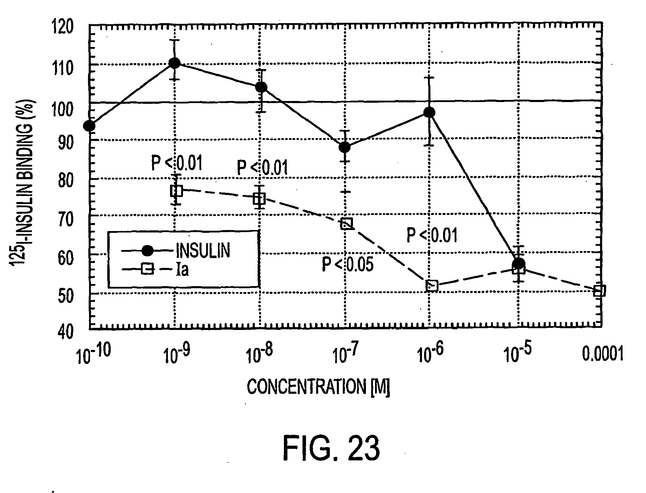

Differentiated 3T3-11 cells (in triplicate wells) were treated with either la or cold insulin for one hour at 37°C at the indicated concentrations. After incubation, excess compounds were washed away, and the cells were incubated with a fixed amount of ¹²⁵I-insulin [10 pM; 2000 Ci/mmol) for 12 hours at 4°C. The cells were washed and then lysed with 0.1 % SDS and counted in a scintillation counter. As expected, increasing the dose of cold insulin inhibited the binding of radioactive insulin, while a 45% inhibition occurred with pre-incubation with la. Results shown in FIG. 23.

### Example 28 (outside the scope of the present invention)

Real-time direct binding of la to the insulin receptor was demonstrated by using a Biocore 3000 [which measures surface plasmon resonance). The intensity and wavelength of light reflected off a metal surface with a thin film of solution on it is affected by the mass concentration of components at the liquid-surface interface. The interaction of molecules in the liquid phase alters the intensity of the reflected light at a particular angle. In this experiment purified insulin receptors containing both alpha and beta subunits were immobilized into the sensor surface of flow cell 2 of a Biocore 3000 with a gold film: flow cell 1 was used as a control for background. When la was injected at concentrations of 200 µM, 100 µM and 10 µM, a response indicative of binding to insulin receptor (binding curve) was seen within a few seconds, which is similar to the binding curves obtained with insulin.

### Example 29 (outside the scope of the present invention)

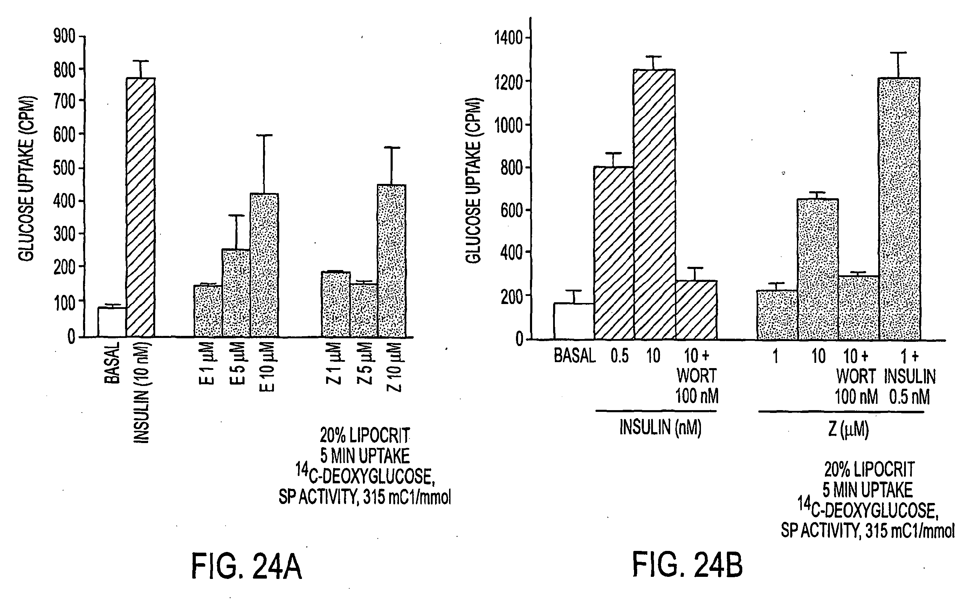

Glucose uptake was measured in normal adipocytes freshly prepared from the epididimal fat pad of SD rats in the presence of E or Z isomers of la or 1b. After the cells were preincubated with the isomers at the indicated concentrations for 30 min. ¹⁴C-deoxy glucose was added, and the preparations were incubated for an additional 5 min. FIG. 24A shows the extent of glucose uptake stimulated by the two isomers was similar. FIG. 24B shows the stimulatory effect of the Z form (Ib) was additive to that of insulin, and the effect was blocked by Wortmannin (15minute preincubation), as was shown earlier for the E form of (la) (see Figure 16).

### Example 30 (outside the scope of the present invention)

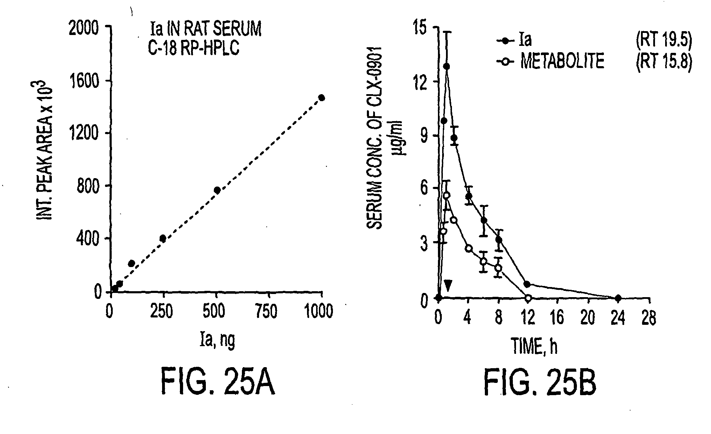

A highly sensitive method for detecting la in Sprague-Dawley (SD) rat serum was developed in which la can be detected at a level of 10-25 ng. The kinetics of drug absorption and clearance from the circulation were studied in a rat model. SD rats were given oral doses of la (20 mg/kg). At different time intervals, blood was collected and serum was analyzed for IA. As shown in the figure, 1a was absorbed maximally at 1 hour following oral delivery of the drug and is cleared from the circulation by 24 hours. Results shown in FIGS. 25A, B.

### Example 31 (outside the scope of the present invention)

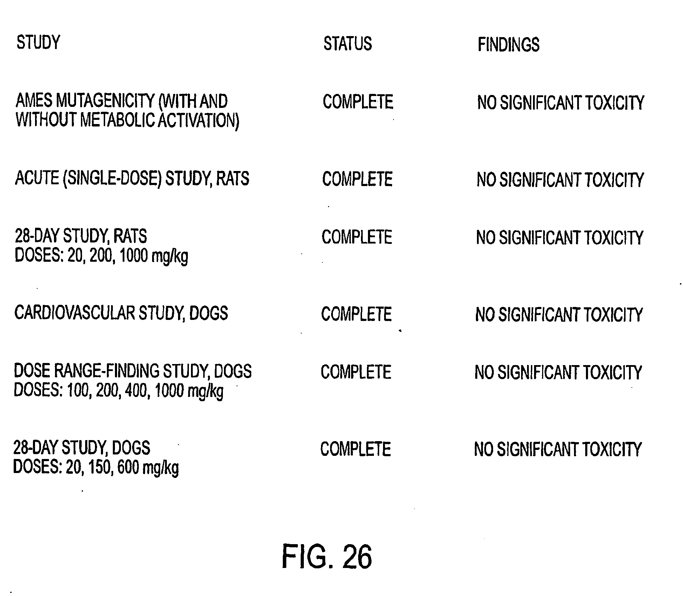

Various toxicology studies have been conducted, and their status and results are summarized in FIG. 26. Oases as high as 1000 mg/kg have been administered, and no serious toxicity issues have been uncovered.

## Claims

1. A compound selected from 3-(3,4-dimethoxy-phenyl)-2-(4-hydroxy-phenyl)-acrylic acid; 3-(3,5-dimethoxy-phenyl)-2-(4-fluoro-p-phenyl)-acrylic acid; 2-(4-acetylamino-phenyl)-3-(3,5-dimethoxy-phenyl)-acrylic acid; or 3-(3,4-dimethoxyphenyl)-2-(4-hydroxy-phenyl)-propionic acid.

2. A pharmaceutical composition for the treatment of diabetes comprising a therapeutically effective amount of a compound, or a mixture of compounds, selected from the group consisting of:
3-(3,4-dimethoxy-phenyl)-2-(4-hydroxy-phenyl)-acrylic acid;
3-(3,5-dimethoxy-phenyl)-2-(4-fluoro-p-phenyl)-acrylic acid;
2-(4-acetylamino-phenyl)-3-(3,5-dimethoxy-phenyl)-acrylic acid; or
3-(3,4-dimethoxy-phenyl)-2-(4-hydroxy-phenyl)-propionic acid;
in a physiologically acceptable carrier.

3. Use of a compound or a mixture of compounds selected from the group consisting of:
3-(3,4-dimethoxy-phenyl)-2-(4-hydroxy-phenyl)-acrylic acid;
3-(3,5-dimethoxy-phenyl)-2-(4-fluoro-p-phenyl)-acrylic acid;
2-(4-acetylamino-phenyl)-3-(3,5-dimethoxy-phenyl)-acrylic acid; or
3-(3,4-dimethoxy-phenyl)-2-(4-hydroxy-phenyl)-propionic acid
in the preparation of a medicament for treating diabetes.

4. The use according to claim 3, wherein the medicament is an oral medicament.

## Patentansprüche

1. Verbindung, die aus 3-(3,4-Dimethoxyphenyl)-2-(4-hydroxyphenyl)acrylsäure; 3-(3,5-Dimethoxyphenyl)-2-(4-fluor-p-phenyl)acrylsäure; 2-(4-Acetylaminophenyl)-3-(3,5-dimethoxyphenyl)acrylsäure und 3-(3,4-Dimethoxyphenyl)-2-(4-hydroxyphenyl)-propionsäure ausgewählt ist.

2. Pharmazeutische Zusammensetzung für die Behandlung von Diabetes, umfassend eine therapeutisch wirksame Menge einer Verbindung oder eines Gemisches von Verbindungen, ausgewählt aus der Gruppe, bestehend aus
3-(3,4-Dimethoxyphenyl)-2-(4-hydroxyphenyl)acrylsäure;
3-(3,5-Dimethoxyphenyl)-2-(4-fluor-p-phenyl)acrylsäure;
2-(4-Acetylaminophenyl)-3-(3,5-dimethoxyphenyl)acrylsäure und
3-(3,4-Dimethoxyphenyl)-2-(4-hydroxyphenyl)propionsäure
in einem physiologisch verträglichen Träger.

3. Verwendung einer Verbindung oder eines Gemisches von Verbindungen, ausgewählt aus der Gruppe, bestehend aus
3-(3,4-Dimethoxyphenyl)-2-(4-hydroxyphenyl)acrylsäure;
3-(3,5-Dimethoxyphenyl)-2-(4-fluor-p-phenyl)acrylsäure;
2-(4-Acetylaminophenyl)-3-(3,5-dimethoxyphenyl)acrylsäure und
3-(3,4-Dimethoxyphenyl)-2-(4-hydroxyphenyl)propionsäure
bei der Herstellung eines Medikaments zur Behandlung von Diabetes.

4. Verwendung nach Anspruch 3, wobei das Medikament ein orales Medikament ist.

## Revendications

1. Composé choisi parmi l'acide 3-(3,4-diméthoxy-phényl)-2-(4-hydroxy-phényl)-acrylique ; l'acide 3-(3,5-diméthoxy-phényl)-2-(4-fluoro-p-phényl)-acrylique ; l'acide 2-(4-acétylamino-phényl)-3-(3,5-diméthoxy-phényl)-acrylique ; ou l'acide 3-(3,4-diméthoxy-phényl)-2-(4-hydroxy-phényl)-propionique.

2. Composition pharmaceutique pour le traitement du diabète, comprenant une quantité thérapeutiquement efficace d'un composé, ou d'un mélange de composés, choisi dans le groupe constitué par
l'acide 3-(3,4-diméthoxy-phényl)-2-(4-hydroxy-phényl)-acrylique ;
l'acide-3-(3,5-diméthoxy-phényl)-2-(4-fluoro-p-phényl)-acrylique ;
l'acide 2-(4-acétylamino-phényl)-3-(3,5-diméthoxy-phényl)-acrylique ; ou
l'acide 3-(3,4-diméthoxy-phényl)-2-(4-hydroxy-phényl)-propionique ;
dans un support physiologiquement acceptable.

3. Utilisation d'un composé, ou d'un mélange de composés, choisi dans le groupe constitué par
l'acide 3-(3,4-diméthoxy-phényl)-2-(4-hydroxy-phényl)-acrylique ;
l'acide 3-(3,5-diméthoxy-phényl)-2-(4-fluoro-p-phényl)-acrylique ;
l'acide 2-(4-acétylamino-phényl)-3-(3,5-diméthoxy-phényl)-acrylique ; ou
l'acide 3-(3,4-diméthoxy-phényl)-2-(4-hydroxy-phényl)-propionique ;
pour la préparation d'un médicament destiné à traiter le diabète.

4. Utilisation selon la revendication 3, dans laquelle le médicament est un médicament oral.
